# EUROPEAN PATENT APPLICATION

(11) **EP 4 495 118 A1**
(43) Date of publication of application: **22.01.2025**
(21) Application number: 23769816.2
(22) Date of filing: 15.03.2023
(51) Int. Cl.: C07D 471/04, A61K 31/519, A61P 17/14

(54) **USE AND TREATMENT METHOD OF AZETIDINE DERIVATIVE**

(30) Priority: 18.03.2022 CN 202210274554
(71) Applicant: Sichuan Kelun-Biotech Biopharmaceutical Co., Ltd., Chengdu, Sichuan 611138 (CN)
(72) Inventor: GE, Junyou, Chengdu, Sichuan 611138 (CN); JIN, Xiaoping, Chengdu, Sichuan 611138 (CN); OUYANG, Xuenong, Chengdu, Sichuan 611138 (CN); CAI, Shirley Xiaoli, Chengdu, Sichuan 611138 (CN); CHENG, Yezhe, Chengdu, Sichuan 611138 (CN); CHENG, Meiling, Chengdu, Sichuan 611138 (CN); HE, Chenghua, Chengdu, Sichuan 611138 (CN)
(74) Representative: Hoffmann Eitle
(86) International application number: PCT/CN2023/081478
(87) International publication number: WO 2023/174314

(57) **Abstract**

Provided is the use of a compound of formula I, a pharmaceutically acceptable salt, a stereoisomer, a polymorph, a solvate, a metabolite or a prodrug thereof, or a composition thereof in the preparation of a drug for treating alopecia such as alopecia areata. An azetidine derivative has clear action mechanisms and pharmacodynamic effects, good pharmacokinetic properties and a good safety, which greatly improves the medication compliance of patients.

## Description

### FIELD OF THE INVENTION

The present invention relates to use of an azetidine derivative and a pharmaceutical composition comprising the same as a Janus kinase (JAK) inhibitor in the treatment of alopecia such as alopecia areata.

### BACKGROUND OF THE INVENTION

Alopecia areata (AA) is a common inflammatory non-scarring alopecia. Clinically, it manifests as sudden, well-defined, round patches of hair loss on the scalp. While mild cases often resolve spontaneously, about half of the patients experience recurrent episodes, which may persist for years or even decades. In severe cases, the condition may affect the entire scalp or even all body hair. AA can occur at any age, but is more common in young and middle-aged individuals, with no significant gender difference.

The pathogenesis of AA is not fully understood. It is currently believed that in AA patients, the upregulation of MHC I and NKG2D on follicular epithelial cells disrupts follicular immune privilege, leading to activation of CD8+ NKG2D+ T cells which attack anagen hair follicles. IL-15 secreted by the hair follicles activates T cells via binding to JAK1 and JAK3, then stimulates the production of IFN-γ. IFN-γ binds to receptors on follicular cells, further triggering IL-15 secretion and forming the positive feedback loop, which contributes to excessive T cells proliferation and facilitating the autoimmune attack on hair follicles. Following that, AA has been developed.

The goal of AA treatments is to control disease progression, promote hair regrowth, prevent and/or reduce relapse, and improve patients' quality of life. Current treatments include: local therapies such as topical corticosteroids, intralesional corticosteroid injections, topical immunotherapy, and topical minoxidil; and systemic therapies such as corticosteroids and immunosuppressants. Recently, studies at home and abroad have reported that some new drugs and treatment methods have certain efficacy in AA, including oral JAK inhibitors, antihistamines (such as ebastine and fexofenadine), compound glycyrrhizin, topical prostaglandin analogues, and treatments involving psoralen combined with long-wavelength ultraviolet radiation (PUVA), narrowband ultraviolet B radiation (UVB), 308 nm excimer lasers, low-energy lasers, and local cryotherapy. However, the efficacy and safety of these treatments need further evaluation.

Therefore, there is a need to develop drugs with better efficacy and safety for the treatments of alopecia, such as alopecia areata.

### SUMMARY OF THE INVENTION

In view of deficiencies in the prior art and actual needs, the object of the present invention is to provide use of a compound of Formula I, a pharmaceutically acceptable salt, stereoisomer, polymorph, solvate, metabolite or prodrug thereof in the manufacture of a medicament for treating alopecia such as alopecia areata. With clear mechanism, certain pharmacological effects, and good pharmacokinetic and safety properties, it greatly improves patient compliance.

To achieve this object, the present invention adopts the following technical solutions.

The present invention provides use of a compound of Formula I, a pharmaceutically acceptable salt, stereoisomer, polymorph, solvate, metabolite or prodrug thereof in the manufacture of a medicament for treating alopecia such as alopecia areata, wherein the compound has the structure represented by Formula I: 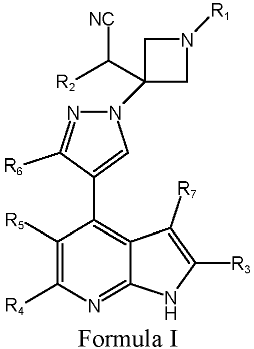 wherein:
R₁ is selected from C(O)R₈ and S(O)₂R₉;
R₂ is selected from H, CN, halogen and C₁₋₆ alkyl;
R₃ and R₄ are each independently selected from H, halogen, and CN;
R₅, R₆ and R₇ are each independently selected from H, halogen, CN, C₁₋₆ alkyl, C₁₋₆ alkoxy, and C(O)NR₁₀R₁₁;
R₈ and R₉ are each independently selected from C₁₋₆ alkyl, C₃₋₁₀ cycloalkyl, 3-10 membered heterocyclyl, C₆₋₁₄ aryl, 5-14 membered heteroaryl, C₇₋₂₀ arylalkyl, and NR₁₀R₁₁;
R₁₀ and R₁₁, at each occurrence, are each independently selected from H and C₁₋₆ alkyl;
wherein the above alkyl, cycloalkyl, heterocyclyl, aryl, heteroaryl and aralkyl are each optionally substituted with 1, 2 or 3 substituents independently selected from halogen, CN, and C₁₋₄ alkyl.

In some embodiments, in the compound of Formula I, R₂ is selected from H, CN, F, and methyl.

In preferred embodiments, in the compound of Formula I, R₂ is selected from H and methyl.

In some embodiments, in the compound of Formula I, R₃ and R₄ are each independently selected from H, F, Cl, and CN.

In preferred embodiments, in the compound of Formula I, R₃ and R₄ are H.

In some embodiments, in the compound of Formula I, R₅, R₆ and R₇ are each independently selected from H, F, Cl, CN, methyl, ethyl, methoxy, and C(O)NH₂.

In preferred embodiments, in the compound of Formula I, R₅ is H, F, Cl, CN, methyl or C(O)NH₂;
R₆ is H, Cl, CN, methyl or methoxy; and
R₇ is H.

In some embodiments, in the compound of Formula I, R₈ and R₉ are each independently selected from methyl, ethyl, n-propyl, isopropyl, cyclopropyl, n-butyl, isobutyl, tert-butyl, aziridinyl, pyrrolidinyl, phenyl, benzyl and N(CH₃)₂, wherein the above groups are each optionally substituted with 1, 2 or 3 substituents independently selected from F, CN and methyl.

In some embodiments, in the compound of Formula I, R₁₀ and R₁₁, at each occurrence, are each independently selected from H, methyl, and ethyl.

In the use encompassed by the present invention, any combination of the above preferred groups may be carried out to obtain the compound of Formula I.

In some embodiments, the invention provides use of a compound, a pharmaceutically acceptable salt, stereoisomer, polymorph, solvate, metabolite or prodrug thereof in the manufacture of a medicament for treating alopecia such as alopecia areata, wherein the compound is selected from:

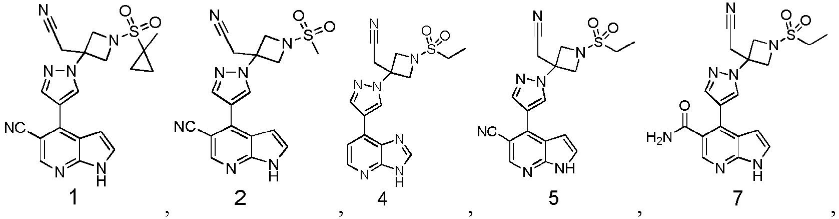

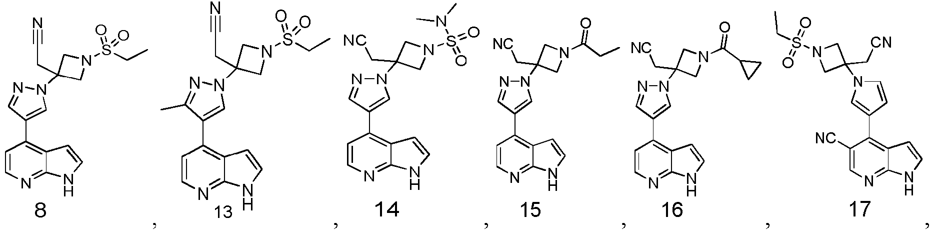

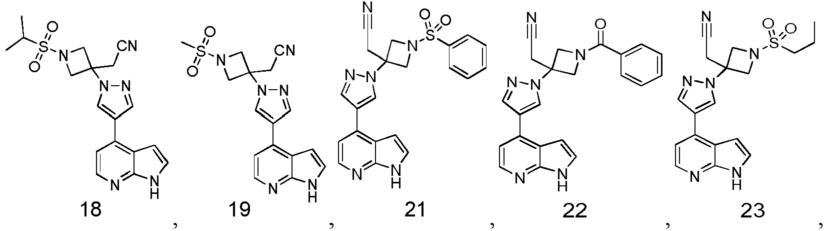

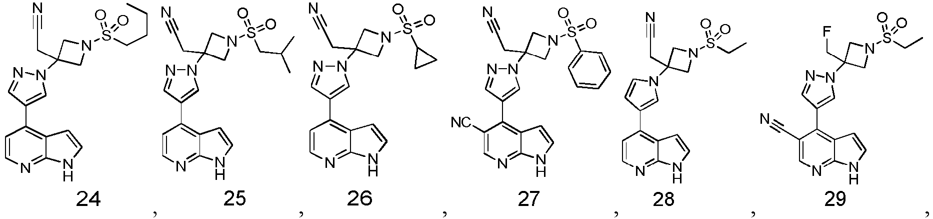

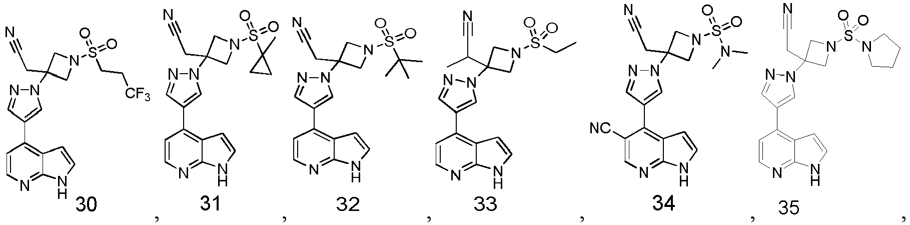

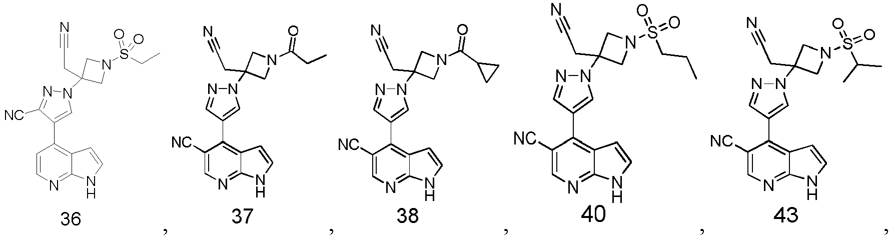

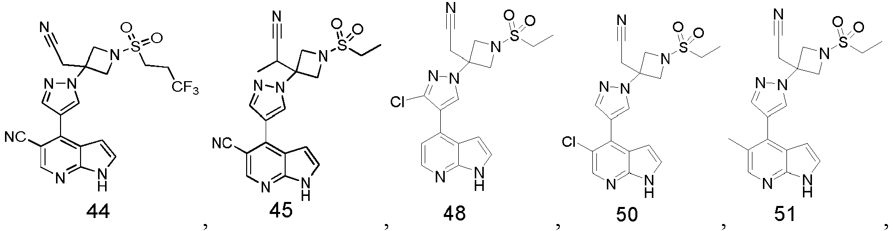

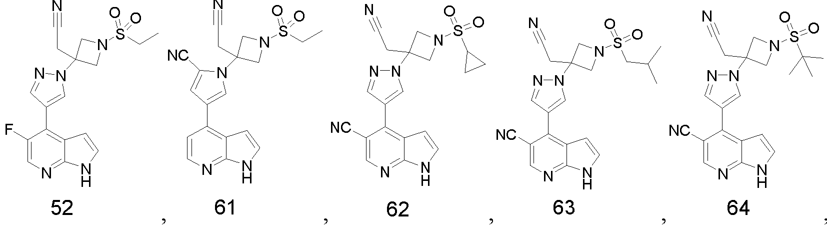

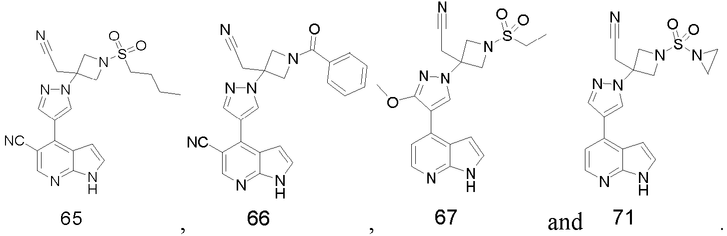

In some embodiments, the compound of Formula I is:

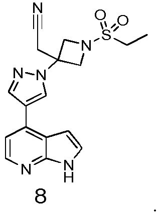

In some embodiments, the medicament of the present invention further comprises a pharmaceutically acceptable excipient.

In some embodiments, the medicament of the present invention further comprises one or more additional therapeutic agents.

In preferred embodiments, the excipient includes any one or a combination of two or more of excipients, diluents, carriers, flavoring agents, binders, or fillers; preferably, the carriers include liposomes, micelles, microspheres, or microcapsules, *etc.*

In some embodiments, the dosage form of the medicament of the present invention includes tablet, capsule, granule, powder, or injection. Each dosage form can be prepared according to conventional methods in the pharmaceutical field.

In some embodiments, the dosage form of the medicament of the present invention is a capsule.

In some embodiments, the dosage form of the medicament of the present invention is a capsule, wherein the specification of the capsule (i.e., the amount of the compound of Formula I, a pharmaceutically acceptable salt, stereoisomer, polymorph, solvate, metabolite or prodrug thereof comprised in each capsule) is 0.1-50 mg, such as 0.1-20 mg, e.g., 0.2-10 mg, e.g., 0.5 mg, 1 mg, 1.5 mg, 2 mg, 2.5 mg, 3 mg, 3.5 mg, 4 mg, 4.5 mg, 5 mg, 5.5 mg or 6 mg, e.g., 0.5 mg, 1 mg or 2 mg.

In some embodiments, the medicament of the present invention can be administered through a suitable route, such as injection, intravenous, intraarterial, subcutaneous, intraperitoneal, intramuscular, or transdermal administration; or through oral, buccal, nasal, transmucosal, topical administration, in the form of an ophthalmic formulation or by inhalation.

In some embodiments, the alopecia of the present invention is inflammatory non-scarring alopecia.

In some embodiments, the alopecia of the present invention is alopecia areata, preferably severe alopecia areata.

The present invention provides a compound of Formula I, a pharmaceutically acceptable salt, stereoisomer, polymorph, solvate, metabolite or prodrug thereof, or a composition comprising the compound, a pharmaceutically acceptable salt, stereoisomer, polymorph, solvate, metabolite or prodrug thereof, for use in treating alopecia, preferably for use in treating alopecia areata, more preferably for use in treating severe alopecia areata.

The present invention provides a method for treating alopecia, wherein the method comprises administering to a subject a therapeutically effective amount of the compound of Formula I as described above, a pharmaceutically acceptable salt, stereoisomer, polymorph, solvate, metabolite or prodrug thereof, or a composition comprising the compound, a pharmaceutically acceptable salt, stereoisomer, polymorph, solvate, metabolite or prodrug thereof. Preferably, the method is for treating alopecia areata, more preferably for treating severe alopecia areata.

In some embodiments, the method includes restoring the normal growth cycle of hair follicles, inducing the proliferation and differentiation of hair follicle stem cells, and/or promoting the proliferation and differentiation of hair follicle stem cells and angiogenesis.

In some embodiments, the method comprises administering to a subject the compound of Formula I, a pharmaceutically acceptable salt, stereoisomer, polymorph, solvate, metabolite or prodrug thereof, or a composition comprising the compound, a pharmaceutically acceptable salt, stereoisomer, polymorph, solvate, metabolite or prodrug thereof once or multiple times daily for 1-60 consecutive weeks, such as 12 weeks, 24 weeks, or 36 weeks. Preferably, it is administered once daily for 36 consecutive weeks.

In some embodiments, at screening or baseline stage, alopecia areata of the subject is measured by the Severity of Alopecia Tool (SALT), and hair loss area is ≥50% of the entire scalp, including alopecia totalis and alopecia universalis, with no spontaneous improvement in the past 6 months (i.e., spontaneous decrease in the SALT score ≤10%), and the alopecia areata duration (time from the last time without scalp alopecia to randomized grouping) is ≤8 years.

In some embodiments, the subject's SALT score (percentage of alopecia) at week 24 or week 36 after the start of treatment is ≤20, e.g., ≤10.

In some embodiments, the subject's SALT score (percentage of alopecia) at week 24 or week 36 after the start of treatment shows at least 50%, improvement compared to baseline, e.g., 70%, 75%, 80%, 85% or 90%.

In some embodiments, the subject's eyebrow assessment (EBA) and eyelash assessment (ELA) at week 24 or week 36 after the start of treatment show at least 2-grade improvement compared to baseline or a score of 3.

In some embodiments, the score of the subject's alopecia scale (Clinician-Reported Outcome, ClinRO) at week 24 or week 36 after the start of treatment is 0 or 1 and the improvement is ≥2 points.

In some embodiments, the score of the subject's global impression scale of disease change (Patient-Reported Outcomes, PRO) at week 24 or week 36 after the start of treatment is 0 or 1.

In some embodiments, the subject's plasma drug concentration before the first administration is less than 5% of Cₘₐₓ.

In some embodiments, the subject should not have any of the following characteristics before the first administration:
(1) Hemoglobin <11.0 g/dL (110.0 g/L);
(2) Neutrophil count (NEUT#), white blood cell count (WBC) < lower limit of normal (LLN);
(3) Alanine aminotransferase (ALT), aspartate aminotransferase (AST) > 2 times the upper limit of normal (2ULN), total bilirubin (TBIL) > 1.5 times the upper limit of normal (1.5ULN); or
(4) eGFR calculated based on Cockcroft-Gault ≤ 60 ml/min or currently undergoing regular hemodialysis.

### DETAILED DESCRIPTION OF THE INVENTION

### Term Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

All patents, published patent applications, publications, references, and other materials mentioned herein are incorporated by reference in their entirety.

Unless otherwise expressly indicated by context, the term "or" as used herein means the term "and/or" and may be used interchangeably with the term "and/or".

As used herein, the term "alkyl" is defined to include saturated aliphatic hydrocarbon comprising straight and branched chains. In some embodiments, alkyl has 1-6, e.g., 1-4, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, tert-butyl, n-pentyl, or n-hexyl), which is optionally substituted with one or more (e.g., 1 to 3) suitable substituents such as halogen (e.g., CF₃, C₂F₅, CHF₂, CH₂F, CH₂CF₃, CH₂Cl or - CH₂CH₂CF₃, *etc.*)*.* The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or *tert*-butyl).

As used herein, the term "alkoxy" refers to a linear, branched or cyclic, saturated monovalent hydrocarbon residue represented by a formula of -O-alkyl, wherein the term "alkyl" is as defined above or refers to a "cycloalkyl" as defined below, such as methoxy, ethoxy, n-propoxy, isopropoxy, cyclopropyloxy, n-butoxy, isobutoxy, tert-butoxy, sec-butoxy, cyclobutoxy, pentoxy, iso-pentoxy or n-hexyloxy group, or isomers thereof.

As used herein, the term "cycloalkyl" refers to a saturated or unsaturated, non-aromatic monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0]nonyl, decahydronaphthalene, *etc*.)), which is optionally substituted with one or more (e.g., 1 to 3) suitable substituents. The cycloalkyl has 3 to 15 carbon atoms. For example, the term "C₃₋₁₀ cycloalkyl" refers to a saturated or unsaturated, non-aromatic monocyclic or polycyclic (e.g., bicyclic) hydrocarbon ring having 3 to 10 ring forming carbon atoms (e.g., cyclopropyl, cyclobutyl, cyclopentyl, cyclohexyl or bicyclo[1.1.1]pentyl), which is optionally substituted with one or more (e.g., 1 to 3) suitable substituents, e.g., methyl substituted cyclopropyl.

As used herein, the term "heterocyclyl" refers to a saturated or unsaturated, monovalent, monocyclic or bicyclic residue having 2, 3, 4, 5, 6, 7, 8 or 9 carbon atoms and one or more (e.g., 1, 2, 3 or 4) heteroatom-containing groups selected from the group consisting of C(=O), O, S, S(=O), S(=O)₂, and NR^{a} wherein R^{a} represents a hydrogen atom, C₁₋₆ alkyl, or C₁₋₆ haloalkyl group, in the ring. A heterocycloalkyl may be linked to the rest of a molecule through any one of the carbon atoms or a nitrogen atom (if present). In particular, 3- to 10-membered heterocyclyl refers to a group having 3 to 10 carbon atoms and heteroatom(s) in the ring, such as, but are not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl or trithianyl.

As used herein, the term "aryl" refers to an all-carbon monocyclic or fused-ring polycyclic aromatic group having a conjugated π electron system. For example, as used herein, the term "C₆₋₁₄ aryl" refers to an aromatic group containing 6 to 14 carbon atoms, such as phenyl or naphthyl. Aryl is optionally substituted with one or more (such as 1 to 3) suitable substituents.

As used herein, the term "heteroaryl" refers to a monovalent monocyclic, bicyclic or tricyclic aromatic ring system having 5, 6, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same or different. Moreover, in each case, it can be benzo-fused. In particular, heteroaryl is selected from the group consisting of thienyl, furyl, pyrrolyl, oxazolyl, thiazolyl, imidazolyl, pyrazolyl, isoxazolyl, isothiazolyl, oxadiazolyl, triazolyl, thiadiazolyl *etc.,* and benzo derivatives thereof; or pyridinyl, pyridazinyl, pyrimidinyl, pyrazinyl, triazinyl, *etc.,* and benzo derivatives thereof.

The term "aralkyl" preferably means aryl substituted alkyl, wherein aryl and alkyl are as defined herein. Normally, the aryl group may have 6-14 carbon atoms, and the alkyl group may have 1-6 carbon atoms. Exemplary aralkyl group includes, but is not limited to, benzyl, phenylethyl, phenylpropyl, phenylbutyl.

As used herein, the term "halo" or "halogen" is defined to include F, Cl, Br, or I.

The term "substituted" means that one or more (e.g., one, two, three, or four) hydrogens on a designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

The term "optionally substituted" means that a compound is optionally substituted with a specified group, radical or moiety.

When a bond to a substituent is shown to cross a bond connecting two atoms in a ring, then such substituent may be bonded to any of the ring-forming atoms in that ring that are substitutable.

The compound of the present invention may also contain one or more (e.g., 1, 2, 3, or 4) isotopes. For example, in the compound of the present invention, hydrogen or H may be in any isotopic form, including ¹H, ²H (D or deuterium) and ³H (T or tritium); carbon or C may be in any isotopic form, including ¹²C, ¹³C and ¹⁴C; and oxygen or O may be in any isotopic form, including ¹⁶O and ¹⁸O, *etc.*

The term "stereoisomer" refers to isomers formed due to the presence of at least one asymmetric center. A compound having one or more (e.g., one, two, three or four) asymmetric centers can give rise to a racemate, racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, solvate, metabolite or prodrug, which can directly or indirectly provide the compound of the present invention or a metabolite or residue thereof after being administered to a patient in need thereof.

A pharmaceutically acceptable salt of the compound of the present invention includes an acid addition salt and a base addition salt thereof.

A suitable acid addition salt is formed from an acid which forms a non-toxic salt. Specific examples include aspartate, bicarbonate/carbonate, bisulfate, borate, camphorsulfonate, citrate, cyclamate, edisylate, esylate, fumarate, gluceptate, gluconate, glucuronate, hexafluorophosphate, hydroiodide/iodide, isethionate, lactate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, tannate, and xinofoate salts.

A suitable base addition salt is formed from a base which forms a non-toxic salt. Specific examples include aluminum, arginine, benzathine, calcium, choline, diethylamine, diolamine, glycine, lysine, magnesium, meglumine, olamine, potassium, sodium, tromethamine and zinc salts.

For a review on suitable salts, see "Hand book of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

The compound of the present invention may exist in the form of a hydrate or a solvate, wherein the compound of the present invention contains a polar solvent, in particular water, methanol or ethanol, for example, as a structural element of the crystal lattice of the compound. Polar solvents, in particular water, may exist in a stoichiometric or non-stoichiometric amount.

The present invention further encompasses a metabolite of the compound of the present invention, i.e., a compound generated *in vivo* upon administration of a drug.

Prodrugs of the compound of the present invention can be formed through replacing suitable functional groups in the compound of Formula I with those known in the art (for example, the "pro-moieties" described in "Design of Prodrugs" by H. Bundgaard (Elsevier, 1985)).

The term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the composition/medicament of the present invention includes, but is not limited to, sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological saline as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The pharmaceutical composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g., Remington's Pharmaceutical Sciences (1990).

The compound/composition/medicament of the present invention can act systemically and/or topically. To this end, it can be administered through a suitable route, such as through injection, intravenous, intraarterial, subcutaneous, intraperitoneal, intramuscular or transdermal administration, or administered via oral, buccal, nasal, transmucosal, topical, as an ophthalmic formulation, or via inhalation.

For these routes of administration, the compound/composition/medicament of the present invention can be administered in a suitable dosage form.

Such dosage forms include, but are not limited to tablets, capsules, lozenges, hard candies, powders, sprays, creams, salves, suppositories, gels, pastes, lotions, ointments, aqueous suspensions, injectable solutions, elixirs, and syrups.

As used herein, the term "therapeutically effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition can be about 0.01 mg to about 1000 mg, suitably 0.1-500 mg, preferably 0.1-300 mg, more preferably 0.1-150 mg, particularly preferably 0.3-50 mg, e.g., 0.5 mg, 1 mg, 1.5 mg, 2 mg, 4 mg, 10 mg, 25 mg, *etc.*

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g. birds, amphibians, reptiles) and mammals, such as non-human primates, livestock and/or domesticated animals (such as sheep, dog, cat, cow, pig and the like). When the subject refers to a human, the terms "subject" and "patient" are used interchangeably herein.

The term "week ..." refers to a period that includes 5 days before and 5 days after the specified number of week (for example, week 4 +/- 3 days, day 28 +/- 3 days, day 253 +/- 5 days).

The term "baseline" refers to a given parameter or patient condition before treatment.

"One or more additional therapeutic agents" refers to agents with a therapeutic effect administered (including simultaneous (concurrent) or sequential administration in any order) other than the active ingredient of the present invention.

The term adverse event (AE) refers to any adverse medical event occurred to a subject after receiving the test drug, which may manifest as symptoms, signs, diseases, or laboratory abnormalities, but is not necessarily causally related to the test drug.

The term serious adverse event (SAE) refers to an adverse event that meets any of the following criteria:
1) resulting in death;
2) being life-threatening, which means that the subject is at immediate risk of death at the time of the adverse event, not implying that it may result in death if it develops severely in the future;
3) requiring hospitalization or prolongation of hospitalization;
4) resulting in permanent or significant disability/incapacity;
5) resulting in congenital anomaly/birth defect;
6) other important medical events. Events that may not immediately threaten life, result in death, or require hospitalization but, based on appropriate medical judgment, may jeopardize the subject or require medical intervention to prevent one of the above outcomes, are also generally considered serious adverse events.

### Examples

The examples and embodiments described herein are for illustrative purposes only, and those skilled in the art will propose various modifications or changes based on these examples and embodiments, which are all encompassed within the spirit and scope of the present application.

The preparation of the compound of Formula I (such as Compound 8) involved in the present invention can be done according to patent application WO2017/097224A1. A specified amount of Compound 8 was prepared into capsules with suitable excipients.

Eligible subjects were screened and randomly assigned before dosing on Day 1 (D1), with the following tasks completed: 1) inclusion/exclusion criteria; 2) laboratory tests; 3) randomization; 4) AA disease assessment: SALT assessment (photographs required), nail assessment (affected nails photographed), eyebrow assessment (photographs required), eyelash assessment (photographs required), ClinRO severity assessment, quality of life assessment (SF-36); these assessments served as disease baseline information; 5) PK sampling, *etc.*

To ensure balance between groups during randomization, the following random stratification factor based on alopecia severity was set: one stratum for alopecia totalis or alopecia universalis and another for non-alopecia totalis and non-alopecia universalis.

Subjects were randomly assigned to treatment groups (test groups) of capsules of Compound 8 of the present invention with doses of 0.5 mg, 1 mg, and 2 mg, or to the placebo group (control group), with 44 cases in each group. All subjects took oral Compound 8 capsules (or placebo) once daily on an empty stomach, for a total duration of 36 weeks covering Treatment Periods A and B.

Treatment Period A: subjects in the test or placebo groups took oral Compound 8 capsules or placebo once daily on an empty stomach for 24 consecutive weeks.

Treatment Period B: all subjects in the placebo control group in Treatment Period A were randomized in Treatment Period B to the Compound 8 capsule treatment groups (test groups) with doses of 0.5 mg, 1 mg, and 2 mg in approximately a 1:1:1 ratio. They took oral Compound 8 capsules once daily on an empty stomach for 12 consecutive weeks. The treatment plan for patients in the test group during Treatment Period A remained unchanged in Treatment Period B, continuing for 12 weeks.

Follow-up visits were conducted in week 2/day 15±3, week 4/day 29±3, week 8/day 57±3, week 12/day 85±3, week 16/day 127±3, week 20/day 141±3, week 24/day 169±3, week 28/day 197±3, week 32/day 225±3, and week 36/day 253±5.

### Experimental Example 1: Assessment of Alopecia Severity

SALT is an assessment based on the condition of scalp alopecia and is a quantitative method for assessing the severity of AA. The SALT score = 0.18×left side score + 0.18×right side score + 0.4×top of the head score + 0.24×back of the head score. The severity of alopecia tool (SALT) refers to Olsen 2004 (Olsen EA, Hordinsky MK, Price VH, et al. Alopecia areata investigational assessment guidelines Part II [J]. J Am Acad Dermatol, 2004, 51(3): 440-447) and Olsen 2011 (Olsen EA. Investigative guidelines for alopecia areata [J]. Dermatol Ther, 2011, 24: 311-319).

Subjects had a SALT score ≥ 50 during screening and baseline stages, and their scalp photos were taken at each visit period for SALT assessment with wigs be removed.

### Experimental Example 2: Assessment of Nails Affected by Alopecia Areata

For all subjects, the number of nails affected by AA was counted at baseline and subsequent visits. Nails with any of the following changes were recorded: pitting, trachyonychia (nail with a rough surface), longitudinal ridging or stripes, lunula red spots, leukonychia (white lines or spots on the nail plate), onycholysis (separation of the nail plate from the nail bed), brittle nails (easily broken nails), *etc.* Photos of affected nails were taken at the baseline visit and during the study treatment.

### Experimental Example 3: Global Impression Scale (ClinRO)

The Global Impression Scale score (ClinRO) was derived from evaluating the subject's scalp alopecia severity based on the following evaluation criteria: none (no alopecia) was 0, mild alopecia was 1, moderate alopecia was 2, severe alopecia was 3, and very severe or complete alopecia was 4.

### Experimental Example 4: Subject's Global Impression Scale of Disease Change (PRO)

The subject's global impression scale of their AA condition change was evaluated based on the following evaluation criteria: significant improvement was 0, moderate improvement was 1, slight improvement was 2, no change was 3, slight worsening was 4, moderate worsening was 5, and significant worsening was 6.

### Experimental Example 5: Eyelash Assessment (ELA)

An eyelash assessment is a numerical rating scale (NRS) used to characterize eyelash loss. The numerical rating scale ranges from 0 (none) to 3 (normal) as follows.

| Score | Description |
|---|---|
| 0 | No eyelashes |
| | • No upper or lower eyelashes on both the left and right eyes |
| 1 | Very few eyelashes |
| | • Moderate or severely reduced density and/or large gaps on one or both eyelashes |
| 2 | Moderate eyelashes |
| | • The density of the upper eyelashes on both sides is normal, with no gaps, while the lower eyelashes on one or both sides have gaps or decreases in density, or |
| | • Normal density of eyelashes on both sides with short gaps, or |
| | • The density of the eyelashes on one or both sides is slightly reduced, with short or no gaps. |
| 3 | Normal eyelashes |
| | • The density of both upper and lower eyelashes from the near inner eye corner to the near outer eye corner was normal without any gap. |
| Note: | |
| The density of lower eyelashes is usually less than that of upper eyelashes; | |
| Short gaps do not significantly distort the appearance of the eyelashes; | |
| Moderate eyelash scores do not require the presence of lower eyelashes. | |

### Experimental Example 6: Eyebrow Assessment (EBA)

An eyebrow assessment is a numerical rating scale used to characterize eyebrow loss. The numerical rating scale ranges from 0 (none) to 3 (normal) as follows.

| Score | Description |
|---|---|
| 0 | No eyebrows |
| | • No eyebrow hair. |
| 1 | Very few eyebrows |
| | • The hair density of one or both eyebrows is normal or reduced, with large gaps in the eyebrows. |
| | • The hair density of one or both eyebrows is severely reduced, with or without gaps in the eyebrows. |
| 2 | Moderate eyebrows |
| | • Normal eyebrow density with short gaps, not significantly distorting the appearance of the eyebrows, or |
| | • Slight reduction in eyebrow density, with or without short gaps, or |
| | • Moderate reduction in eyebrow density without short gaps. The visual clarity of the eyebrows is 3 feet. |
| 3 | Normal eyebrows |
| | • The hair density of both left and right eyebrows is normal, spanning normal length (i.e., from the middle part of the eyebrows to near the temples) and width, with no gaps. |
| Note: | |
| The density of the lateral part of the eyebrows might be slightly less than that of the medial part. | |
| Short gaps do not significantly distort the appearance of the eyebrows. | |

### Experimental Example 7: Blocking Effect of Compound on JAK/STAT Signaling Pathway

The inhibitory effect of Compound 8 on STAT5 phosphorylation induced by cytokine IL-2 stimulation in CD4+ T cells in human whole blood, as well as on STAT3 phosphorylation induced by cytokine IL-6 stimulation in CD4+ T cells in human whole blood, was detected using flow cytometry.

The results indicated that Compound 8 inhibited STAT5 phosphorylation induced by IL-2 stimulation in CD4+ T cells in human whole blood and STAT3 phosphorylation induced by IL-6 stimulation in CD4+ T cells in human whole blood. The IC₅₀ for STAT5 phosphorylation inhibition was 0.46 ± 0.03 µM, and the IC₅₀ for STAT3 phosphorylation inhibition was 1.51 ± 0.06 µM.

### Experimental Example 8: In Vitro Kinase Inhibition Activity Study

An *in vitro* enzymatic assay system was used to test the inhibitory effect of Compound 8 on certain kinase targets. The results showed that at a concentration of 200 nM, Compound 8 had inhibition rates of 67% on p70S6K (p70 ribosomal protein S6 kinase) and 75% on PKA (protein kinase A), respectively.

The above descriptions merely pertain to the preferred examples of the present invention and are not intended to limit the invention. Any modifications, equivalent replacements, or improvements made within the spirit and principles of the present invention should be included within the scope of protection of the present invention. Additionally, the technical solutions in the various examples can be combined, but they must be based on the premise that those skilled in the art can implement them. When the combination of technical solutions results in contradictions or cannot be realized, it should be considered that such a combination does not exist and is not within the scope of protection claimed by the present invention.

## Claims

1. Use of a compound of Formula I, a pharmaceutically acceptable salt, stereoisomer, polymorph, solvate, metabolite or prodrug thereof in the manufacture of a medicament for treating alopecia such as alopecia areata, wherein the compound has the structure represented by Formula I: 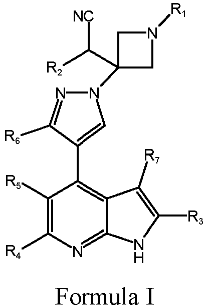 wherein:
R₁ is selected from C(O)R₈ and S(O)₂R₉;
R₂ is selected from H, CN, halogen and C₁₋₆ alkyl;
R₃ and R₄ are each independently selected from H, halogen, and CN;
R₅, R₆ and R₇ are each independently selected from H, halogen, CN, C₁₋₆ alkyl, C₁₋₆ alkoxy, and C(O)NR₁₀R₁₁;
R₈ and R₉ are each independently selected from the group consisting of C₁₋₆ alkyl, C₃₋₁₀ cycloalkyl, 3-10 membered heterocyclyl, C₆₋₁₄ aryl, 5-14 membered heteroaryl, C₇₋₂₀ arylalkyl, and NR₁₀R₁₁;
R₁₀ and R₁₁, at each occurrence, are each independently selected from H and C₁₋₆ alkyl; and
wherein the above alkyl, cycloalkyl, heterocyclyl, aryl, heteroaryl and aralkyl are each optionally substituted with 1, 2 or 3 substituents independently selected from halogen, CN, and C₁₋₄ alkyl.

2. The use of claim 1, wherein said R₂ is selected from H, CN, F and methyl;
preferably, R₂ is selected from H and methyl.

3. The use of claim 1 or 2, wherein the R₃ and R₄ are each independently selected from H, F, Cl, and CN;
preferably, R₃ and R₄ are H.

4. The use of any one of claims 1-3, wherein the R₅, R₆ and R₇ are each independently selected from H, F, Cl, CN, methyl, ethyl, methoxy, and C(O)NH₂;
preferably, R₅ is H, F, Cl, CN, methyl or C(O)NH₂;
R₆ is H, Cl, CN, methyl or methoxy; and
R₇ is H.

5. The use of any one of claims 1-4, wherein the R₈ and R₉ are each independently selected from methyl, ethyl, n-propyl, isopropyl, cyclopropyl, n-butyl, isobutyl, *tert*-butyl, aziridinyl, pyrrolidinyl, phenyl, benzyl and N(CH₃)₂, wherein the above groups are each optionally substituted with 1, 2 or 3 substituents independently selected from F, CN and methyl.

6. The use of any one of claims 1-5, wherein the R₁₀ and R₁₁, at each occurrence, are each independently selected from H, methyl, and ethyl.

7. Use of a compound, a pharmaceutically acceptable salt, stereoisomer, polymorph, solvate, metabolite or prodrug thereof in the manufacture of a medicament for treating alopecia such as alopecia areata, wherein the compound is selected from:

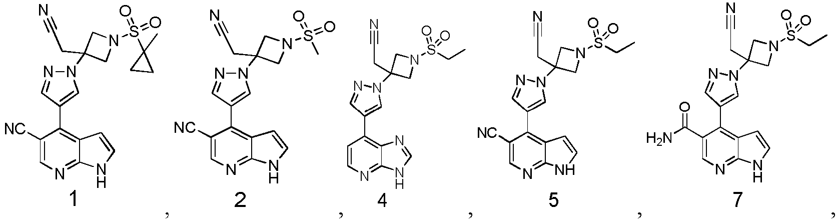

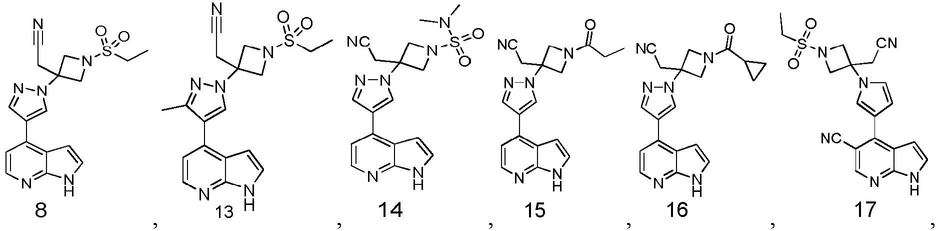

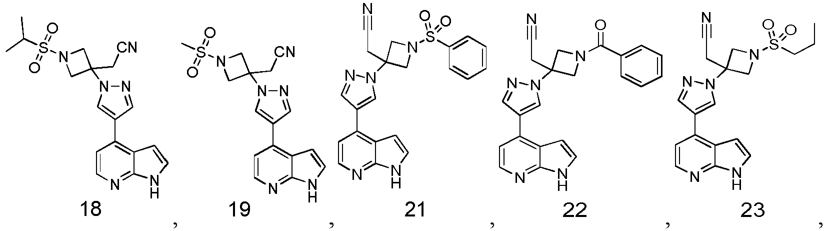

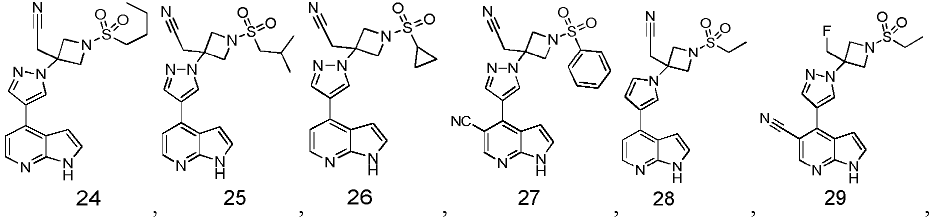

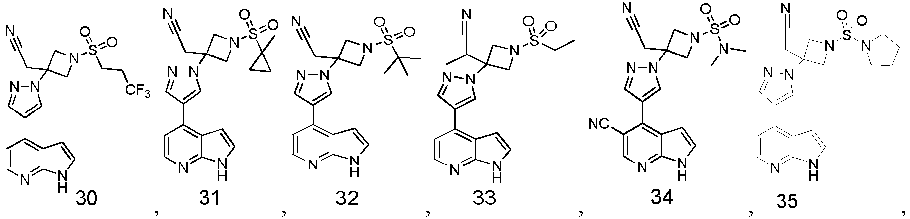

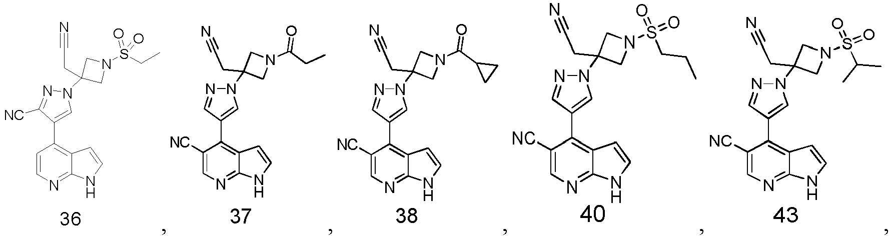

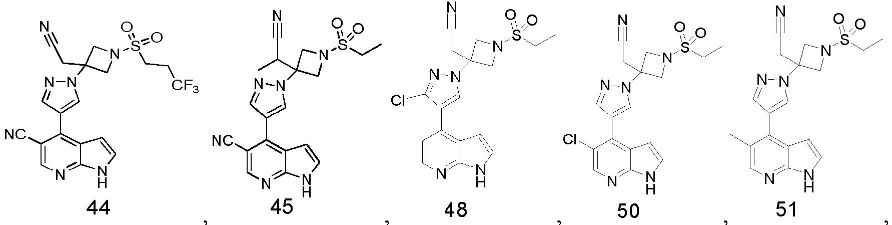

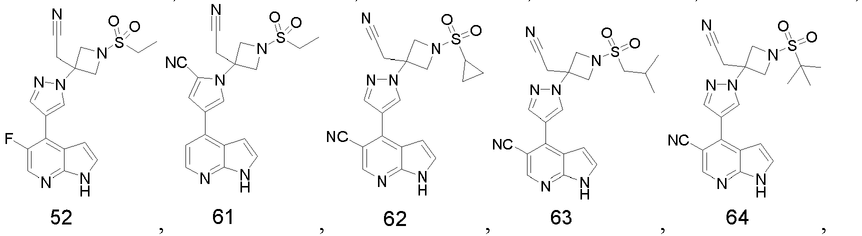

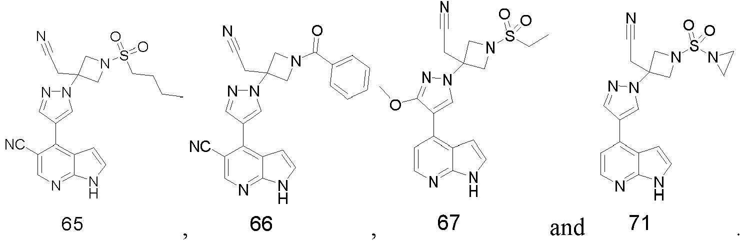

8. The use of any one of claims 1-7, wherein the medicament further comprises a pharmaceutically acceptable excipient.

9. The use of any one of claims 1-8, wherein the dosage form of the medicament includes tablet, capsule, granule, powder, or injection, preferably the dosage form of the medicament is a capsule.

10. The use of claim 9, wherein the specification of the capsule is 0.1-50 mg, such as 0.1-20 mg, e.g., 0.2-10 mg, e.g., 0.5 mg, 1 mg, 1.5 mg, 2 mg, 2.5 mg, 3 mg, 3.5 mg, 4 mg, 4.5 mg, 5 mg, 5.5 mg or 6 mg, e.g., 0.5 mg, 1 mg or 2 mg.

11. The use of any one of claims 1-10, wherein the alopecia is alopecia areata, preferably severe alopecia areata.

12. The use of any one of claims 1-11, further comprising administering to a subject the compound or a composition comprising the same, at a dosing regimen of once or multiple times daily for 1-60 consecutive weeks, such as 12 weeks, 24 weeks, or 36 weeks, preferably, it is administered once daily for 36 consecutive weeks.

13. The use of claim 12, wherein at screening or baseline stage, alopecia areata of the subject is measured by the Severity of Alopecia Tool (SALT), and hair loss area is ≥50% of the entire scalp, including alopecia totalis and alopecia universalis, with no spontaneous improvement in the past 6 months (i.e., spontaneous decrease in the SALT score ≤10%), and the alopecia areata duration (time from the last time without scalp alopecia to randomized grouping) is ≤8 years; or the subject's SALT score (percentage of alopecia) at week 24 or week 36 after the start of treatment is ≤20, e.g., ≤10; or the subject's SALT score (percentage of alopecia) at week 24 or week 36 after the start of treatment shows at least 50% improvement compared to baseline, e.g., 70%, 75%, 80%, 85% or 90%; or the subject's eyebrow assessment (EBA) and eyelash assessment (ELA) at week 24 or week 36 show at least 2-grade improvement compared to baseline or a score of 3; or the score of the subject's alopecia scale (ClinRO) at week 24 or week 36 after the start of treatment is 0 or 1 and the improvement is ≥2 points; or the score of the subject's global impression scale of disease change (PRO) at week 24 or week 36 after the start of treatment is 0 or 1; or the subject's plasma drug concentration before the first administration is less than 5% of Cₘₐₓ; or the subject should not have any of the following characteristics before the first administration:
(1) Hemoglobin <11.0 g/dL (110.0 g/L);
(2) Neutrophil count (NEUT#), white blood cell count (WBC) < lower limit of normal (LLN);
(3) Alanine aminotransferase (ALT), aspartate aminotransferase (AST) > 2 times the upper limit of normal (2ULN), total bilirubin (TBIL) > 1.5 times the upper limit of normal (1.5ULN); or
(4) eGFR calculated based on Cockcroft-Gault ≤ 60 ml/min or currently undergoing regular hemodialysis.
